# EUROPEAN PATENT SPECIFICATION

(11) **EP 0 347 084 B1**
(45) Date of publication and mention of the grant of the patent: **09.03.1994**
(21) Application number: 89305658.0
(22) Date of filing: 05.06.1989
(51) Int. Cl.: A61F 9/00

(54) **Ophthalmic device**
Vorrichtung für Augenheilkunde
Appareil ophtalmique

(30) Priority: 09.06.1988 GB 8813697; 15.02.1989 GB 8903470
(43) Date of publication of application: 20.12.1989
(73) Proprietor: Williams, John Leslie, Kidlington Oxford OX5 2BX (GB)
(72) Inventor: Williams, John Leslie, Kidlington Oxford OX5 2BX (GB)
(74) Representative: Rock, Olaf Colin

(56) References cited:
- FR-A- 1 259 476
- GB-A- 1 280 014
- US-A- 3 446 209
- US-A- 4 531 944
- US-A- 4 733 802

## Description

This invention relates to an ophthalmic device. It is particularly, though not exclusively, concerned with a device whereby a person can readily treat one of their own eyes with a material with reasonable efficiency. The material can be in virtually any material phase (such as a gas, vapour, liquid or powder).

A variety of methods are known for the treatment of eyes which require varying degrees of skill on the part of the user. A skilled practitioner can align the head of a patient and can use a simple dropper to administer the required number of drops of medication swiftly and on a part of the eye which ensures the most effective distribution to the eye. On many occasions it is necessary for patients to undertake self administration of eye treatment such as with drops or ointment. If the patient is a person wanting in self confidence or lacking manipulative skills attempts at self administration can result in ineffective application, if not total wastage, of medication.

For self administration of a liquid an alternative to an eye dropper is an eyebath whereby a liquid medicament is carried to the eye so as to immerse at least a part of the eye. Thereafter the eye is opened and closed to provide for the distribution of medicament over the eye surface. This again requires a degree of manipulative skill and freedom of head action on the part of the user. In addition the method of application is necessarily a rather random affair involving the distribution of a relatively large amount of medication over a relatively large area despite a probable need for the medication to act on a relatively small area.

There is a need for an ophthalmic device whose use results in the efficient application of medication to a selected area of the eye particularly for cases involving self administration.

US Patent 4 531 944 (Bechtle) shows an eye drop application aid for self-application of droplets of ophthalmic solution by an individual including a housing having top, side walls and an open bottom, with the lower periphery of the side walls being contoured to conform to the facial area surrounding the eye socket. The housing top includes an eye drop dispenser seat with a central droplet discharge aperture therethrough for receiving and supporting the dispensing end of an eye drop dispenser. A vertical alignment indicator is mounted on the housing in view of the eye being treated to achieve vertical alignment of the dispenser end before dispensing droplets of ophthalmic solution to insure accurate application of the droplets to the eye. An eye distracting orifice in the housing top in view of the eye being treated focuses the eye's attention on the orifice instead of the dispenser end to avoid premature blinking before the droplet has contacted the eye.

The application aid described in US 4 531 944 is device requiring the user to align their head with the eye vertical. The provision of a vertical alignment indicator in the device between the eye and the distracting orifice results in a bulky aid. In addition the aid has a number of nooks and crannies which can serve to retain bacteria or other foreign matter which could lead to infection and contamination of the aid and of the dropper device by means of which droplets are released into the eye.

Accordingly it is an object of the present invention to provide an ophthalmic device of simple design which is readily applied to the eye of a user to enable them to apply to their eye droplets or other material phases of medication. It is a further object for the device to be of generally open form without crevices so as to be readily sterilised such as by boiling in water. In addition it is a further object to provide a device whereby medicating containers as supplied by a manufacturer are readily attached to the device means so that medication can be dispensed directly from a containment to the eye without a need for an intermediate transfer of medication by a further device such as a dropper.

According to a first aspect of the present invention there is provided an ophthalmic device in the form of a cup having a periphery substantially scaphoid in shape for contacting the facial region in the vicinity of the eye; the second port; the first port providing an inlet for a dispensing unit for material to be supplied to the eye, the second port serving as a target for directing the eye during the application of material to the eye; and a mounting adapted to locate a dispensing outlet of a dispenser retained on or by the mounting at a datum position relative to the mounting; the device being **characterised by** a projection (36, 61) on the device (30, 50) and extending from the periphery (32, 52) in a direction away from the interior of the cup (31, 51), the projection (36, 61) forming a contact point for the device (30, 50) on a portion of a face beneath an eye (E) adapted so as to cause downward displacement with respect to the eye of the skin in the region of the contact point on relative movement between device (30, 50) and the eye (E) as the device is located about the eye.

According to a first preferred version of the present invention the cup is **characterised in that** it comprises a first portion (52) and a second portion (53) linked together by a hinge (54) link whereby the portions (52, 53) can be moved relative to one another between a closed position wherein the device (50) is available for use and an open position wherein a dispenser (63) can be mounted into the first portion (52) by a holder (58).

According to a second preferred version of the present invention or the first preferred version thereof the device is **characterised by** the provision of an aligning sight (N) on the outside of the device (30) so that as the device (30) is moved towards the eye the sight (N) enables the eye to provide for the correct relative alignment of the device (30) immediately prior to contact between the projection (36, 61) and the contact point.

According to a second aspect of the present invention there is provided the combination of an ophthalmic device according to the present invention or any preceding preferred version thereof and a dispenser having a dispensing head **characterised by** the dispenser (38, 63) being mounted in the first port (34, 58, 59) whereby material from the head (39, 64) can be dispensed into the interior of the cup (31, 51) at least when the first portion (52) is in the working position; the hinge (54) and the first portion (52) providing that when the first portion (52) with dispenser (38, 63) mounted thereon moves between the open and the closed positions the head (39, 64) does not strike the device (50).

Exemplary embodiments of the invention will now be described with reference to the accompanying drawings of ophthalmic devices for dispensing medicament of which:
Figure 1 is a front view of a first embodiment;
Figure 2 is an bottom view of the device shown in Figure 1;
Figure 3 is a side view of the device shown in Figure 1; and
Figure 4 is a view of the interior of the device shown in Figure 1.
Figures 5 and 6 are perspective views (Figure 6 being partially in section) of the device shown in Figures 1 to 4.
Figure 7 is a perspective view of a second embodiment;
Figures 8 and 9 are pictorial views of the device shown in Figure 7.

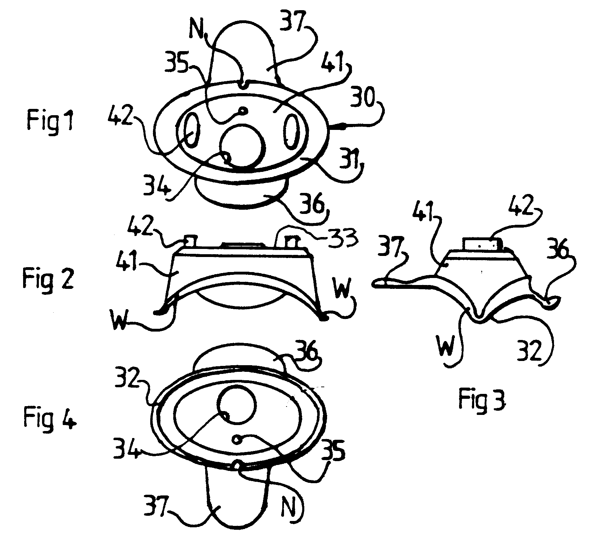

### FIRST EMBODIMENT (Figures 1 to 6)

Device 30 comprises a cup 31 of plastics material having a working periphery 32 which, as variously viewed in Figures 1 to 4, is of scaphoid shape so as to be able to fit onto the face of a person around an eye. The cup has wing sections W on either side. Wall 33 of the cup 31 is pierced by first port 34 and second port 35. The cup has integral components comprising a lip 36 and an extension 37. An alignment notch N is provided on the upper part of the working periphery 32.

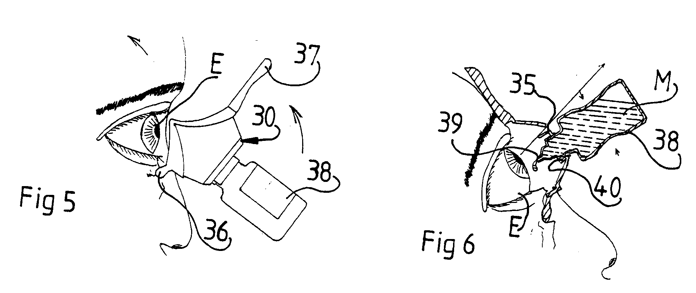

First port 34 is adapted to receive a liquid dispenser 38 (shown only in Figures 5 and 6) having a dispensing head 39 and containing a medicament M. The dispenser 38 is demountably retained by a resilient lip seal 40 integral with the cup 31.

The second port 35 serves as a viewing target so that with device appropriately located to cover the eye E when the eye E is directed to look at the port 35 it is correctly aligned to receive medicament M ejected from dispensing head 39 by squeezing the body of the dispenser 38.

Wall 33 has an outer flat section carrying stubs 42 whereby a regulator (not shown) for the dispenser 38 can be demountably secured to the device.

In use prior to locating the device over the eye E the dispenser 38 is mounted in first port 34 by way of the resilient seal 40. The device 30 is then moved towards the eye which views notch N so as to enable the device to be manipulated to ensure the correct alignment of the device relative to the eye. Eventually, as shown in Figure 5, the lip 36 contacts the top of the cheek below the eye. The device 30 is then moved on towards the eye so that wings W snugly fit around either end of the eye and beneath the eyebrow. In moving into position the device is moved downwardly so causing the lip 36 to move downwardly which causes the lower lid of the eye to be drawn downward slightly so exposing more of the eye ball and facilitating the entry of medicament into the eye. Final location of the device occurs when extension 37 seats on the lower forehead of the user. In this position the second port 35 can be viewed by way of pupil. The head is then thrown back to the position shown in Figure 6 and while the view from the pupil E is directed to the target presented by port 35 the dispenser 38 is squeezed to dispense the required amount of medicament. If necessary the head is turned to ensure that gravity acts in a direction ensuring that dispensed material is distributed appropriately over the eye.

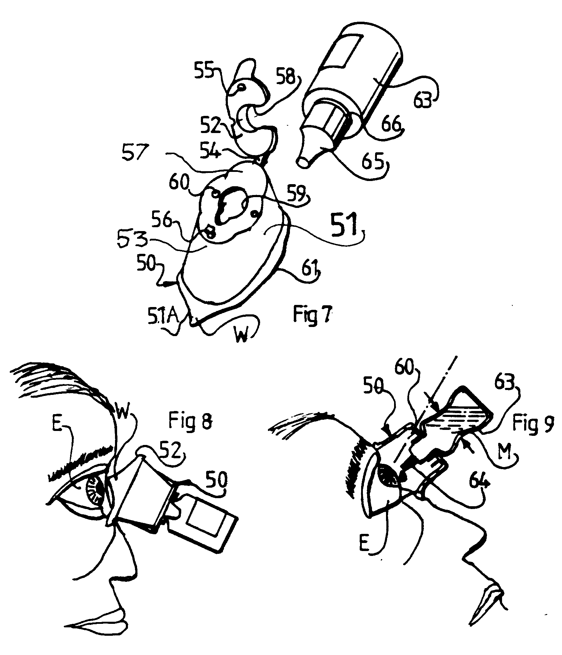

### SECOND EMBODIMENT (Figures 7 to 9)

Device 50 comprises a cup 51 of plastics material having a working periphery 51A which, as variously viewed in Figures 7 and 8, is of scaphoid shape so as to be able to fit onto the face of a person around an eye E. The cup has wing sections W on either side. The cup 51 is made up of a minor first portion 52 and a major second portion 53 which are pivotably connected by way of hinge 54. At the opposite end of first portion 52 to hinge 54 is an aperture 55 which with the first portion 52 in its closed position (as indicated in Figure 8) engages a retaining stud 56 formed in face 57 of second portion 53. The first portion 52 incorporates a crutch 58 which, with the first portion (52) in its closed position, is coaxial with first port 59 in second portion 53. The second portion 53 also contains a second port 60. The cup 51 has an integral lip 61.

Crutch 58 is adapted to demountably receive a liquid dispenser 63 containing medicament M (shown detached in Figure 7 and mounted in Figures 8 and 9) having a dispensing head 64 which when not in use is closed by a conventional cap 65.

The second port 60 serves as a viewing target so that with device 50 appropriately located to cover the eye E when the eye E is directed to look at the port 60 it is appropriately aligned to receive medicament ejected from dispensing head 64 by squeezing the body of the dispenser 63.

In use prior to locating the device 51 over the eye E the first portion 52 of the cup 51 is hinged outwardly from the second portion 53 to expose the first port 59. The (capped) dispenser 63 is then mounted in crutch 58 by sliding gap 66 between the cap 65 and the shoulders of the dispenser 63 so that the closed dispenser is retained in the crutch 58. The cap 65 is then removed from the dispenser 63 to expose the dispensing head 64. The first portion 52 is then closed to cause aperture 55 to engage retaining stud 56. The relative geometry and alignment of the crutch 58, dispensing head 64, and port 59 are such as to ensure a clear passage for the head 64 in passing between the interior of the cup 51 as shown in Figure 8 to the opened position shown in Figure 7. The assembled device 51 is then moved towards the eye until, as shown in Figure 8, the lip 61 contacts the top of the cheek below the eye E. The device 51 is then moved on towards the eye so that wings W snugly fit around either end of the eye and beneath the eyebrow. In moving into this position the device tends to move downwardly causing the lip 61 to move downwardly causing the lower lid of the eye E to be drawn downward slightly so exposing more of the eye ball and facilitating the entry of medicament into the eye. Once the device reaches the position shown in Figure 9 the head is thrown back and the view from the pupil P is directed to the target presented by port 60. The dispenser 63 is squeezed to dispense the requisite amount of medicament M. If necessary the head is turned to ensure that gravity acts in a direction ensuring that dispensed material is distributed appropriately over the eye.

## Claims

1. An ophthalmic device in the form of a cup having a periphery substantially scaphoid in shape for contacting a facial region in the vicinity of an eye; the cup interior being accessible from outside the cup by way of at least a first and a second port; the first port providing an inlet for a dispensing unit for material to be supplied to the eye, the second port serving as a target for directing the eye during the application of material to the eye; and a mounting adapted to locate a dispensing outlet of a dispenser retained on or by the mounting at a datum position relative to the mounting; the device being **characterised by** a projection (36, 61) on the device (30, 50) and extending from the periphery (32, 52) in a direction away from the interior of the cup (31, 51), the projection (36, 61) forming a contact point for the device (30, 50) on a portion of a face beneath an eye (E) adapted to cause downward displacement with respect to the eye of the skin in the region of the contact point on relative movement between device (30, 50) and the eye (E) as the device is located about the eye.

2. An ophthalmic device as claimed in Claim 1 **characterised in that** the cup (51) comprises a first portion (52) and a second portion (53) linked together by a hinge (54) whereby the portions (52, 53) can be moved relative to one another between a closed position wherein the device (50) is available for use and an open position wherein a dispenser (63) can be mounted into the first portion (52) by a holder (58).

3. An ophthalmic device as claimed in any preceding claim **characterised by** the provision of an aligning sight (N) on the outside of the device (30) so that as the device (30) is moved towards the eye the sight (N) enables the eye to provide for the correct relative alignment of the device (30) immediately prior to contact between the projection (36, 61) and the contact point.

4. The combination of an ophthalmic device as claimed in any preceding claim and a dispenser having a dispensing head **characterised by** the dispenser (38, 63) being mounted in the first port (34, 58, 59), whereby material from the head (39, 64) can be dispensed into the interior of the cup (31, 51) at least when the first portion is in the working position; the hinge (54) and the first portion (52) providing that when the first portion (52) with dispenser (38, 63) mounted thereon moves between the open and the closed positions, the head does not strike the device (50).

## Patentansprüche

1. Ophthalmologische Vorrichtung in Form eines Bechers mit einem im wesentlichen wannenförmigen Umfang zum in Berührung Treten mit einem Gesichtsbereich in der Nähe eines Auges; wobei das Becherinnere von außerhalb des Bechers über wenigstens eine erste Öffnung und eine zweite Öffnung zugänglich ist; wobei die erste Öffnung einen Zugang für eine Spendereinheit für dem Auge zuzuführendes Material schafft und die zweite Öffnung als Ziel zum Ausrichten des Auges während der Zufuhr von Material zu dem Auge dient; wobei eine Halterungseinrichtung zum Festlegen einer Spenderauslaßöffnung eines Spenders ausgelegt ist, der an oder durch die Halterungseinrichtung in einer Bezugsposition relativ zu der Halterungseinrichtung festgehalten ist;
wobei die Vorrichtung gekennzeichnet ist durch einen Fortsatz (36, 61) an der Vorrichtung (30, 50), der sich von dem Umfang (32, 52) in Richtung vom Inneren des Bechers (31, 51) weg wegerstreckt, wobei der Fortsatz (36, 61) eine Kontaktstelle für die Vorrichtung (30, 50) an einem Gesichtsbereich unterhalb eines Auges (E) bildet und dazu ausgelegt ist, eine in bezug auf das Auge nach unten gehende Verlagerung der Haut in dem Bereich der Kontaktstelle bei Relativbewegung zwischen der Vorrichtung (30, 50) und dem Auge (E) bei Positionierung der Vorrichtung um das Auge hervorzurufen.

2. Ophthalmologische Vorrichtung nach Anspruch 1,
dadurch gekennzeichnet, daß der Becher (51) einen ersten Bereich (52) und einen zweiten Bereich (53) aufweist, die durch ein Gelenk (54) miteinander verbunden sind, so daß sich die Bereiche (52, 53) zwischen einer geschlossenen Stellung, in der die Vorrichtung (50) für die Verwendung bereit ist, und einer geöffneten Stellung, in der sich ein Spender (63) durch einen Halter (58) in dem ersten Bereich (52) anbringen läßt, relativ zueinander bewegen lassen.

3. Ophthalmologische Vorrichtung nach einem der vorausgehenden Ansprüche,
gekennzeichnet durch das Vorsehen eines Ausrichtungsvisiers (N) an der Außenseite der Vorrichtung (30), so daß bei Bewegung der Vorrichtung (30) in Richtung auf das Auge das Visier (N) dem Auge ermöglicht, für die korrekte relative Ausrichtung der Vorrichtung (30) unmittelbar vor dem Kontakt zwischen dem Fortsatz (36, 61) und der Kontaktstelle zu sorgen.

4. Kombination einer ophthalmologischen Vorrichtung nach einem der vorausgehenden Ansprüche und eines Spenders mit einem Abgabekopf,
dadurch gekennzeichnet, daß der Spender (38, 63) in der ersten Öffnung (34, 58, 59) angebracht ist, wodurch sich Material von dem Kopf (39, 64) ins Innere des Bechers (31, 51) abgeben läßt, mindestens wenn sich der erste Bereich in der Arbeitsstellung befindet, wobei das Gelenk (54) und der erste Bereich (52) dafür sorgen, daß bei Bewegung des ersten Bereichs (52) mit dem daran angebrachten Spender (38, 63) zwischen der geöffneten und der geschlossenen Stellung der Kopf nicht gegen die Vorrichtung (50) stößt.

## Revendications

1. Dispositif ophtalmique sous forme d'une cuvette ayant une périphérie de forme pratiquement scaphoïde, destinée à être au contact d'une région faciale à proximité de l'oeil, l'intérieur de la cuvette étant accessible depuis l'extérieur de la cuvette par au moins un premier et un second orifice, le premier orifice formant une entrée pour un ensemble de distribution de la matière qui doit être transmise à l'oeil, le second orifice étant utilisé comme cible pour diriger l'oeil pendant l'application de la matière à l'oeil, et un organe de montage destiné à positionner une sortie de distribution d'un organe distributeur retenu sur l'organe de montage ou par montage à une position de référence par rapport à l'organe de montage, le dispositif étant caractérisé par une saillie (36, 61) du dispositif (30, 50) partant de la périphérie (32, 52) du côté opposé à l'intérieur de la cuvette (31, 51), la saillie (36, 61) formant un point de contact pour le dispositif (30, 50) avec une partie du visage au-dessous d'un oeil (E) de manière qu'un déplacement vers le bas de la peau de la région du point de contact par rapport à l'oeil soit provoqué lors du déplacement relatif du dispositif (30, 50) et de l'oeil (E) lorsque le dispositif est positionné autour de l'oeil.

2. Dispositif ophtalmique selon la revendication 1, caractérisé en ce que la cuvette (51) comporte une première partie (52) et une seconde partie (53) reliées l'une à l'autre par une articulation (54), de manière que les deux parties (52, 53) puissent être déplacées l'une par rapport à l'autre entre une position de fermeture dans laquelle le dispositif (50) peut être utilisé et une position d'ouverture dans laquelle un distributeur (63) peut être monté dans la première partie (52) par un support (58).

3. Dispositif ophtalmique selon l'une quelconque des revendications précédentes, caractérisé par la disposition d'un organe de visée d'alignement (N) à l'extérieur du dispositif (30) afin que, lorsque le dispositif (30) est déplacé vers l'oeil, l'organe de visée (N) permette à l'oeil d'assurer un alignement relatif convenable du dispositif (30) juste avant contact entre la saillie (36, 61) et le point de contact,

4. Combinaison d'un dispositif ophtalmique selon l'une quelconque des revendications précédentes et d'un distributeur ayant une tête de distribution, caractérisée en ce que le distributeur (38, 63) est monté dans le premier orifice (34, 58, 59) de manière que la matière provenant de la tête (39, 64) puisse être redistribuée à l'intérieur de la cuvette (31, 51) au moins lorsque la première partie est en position de travail, l'articulation (54) et la première partie (52) empêchant le choc de la tête et du dispositif (50) lorsque la première partie (52) portant le distributeur (38, 63) monté sur elle se déplace entre les positions d'ouverture et de fermeture.
